# EUROPEAN PATENT SPECIFICATION

(11) **EP 1 870 120 B1**
(45) Date of publication and mention of the grant of the patent: **02.12.2009**
(21) Application number: 07010038.3
(22) Date of filing: 21.05.2007
(51) Int. Cl.: A61M 1/36, A61J 1/00

(54) **Venous bag in an extracorporeal blood circuit comprising an elastically deformable filament**
Venöser Beutel für einen extrakorporalen Blutkreislauf mit einem elastisch deformierbaren Draht
Poche veineuse dans un circuit sanguin extracorporel avec filament déformable élastiquement

(30) Priority: 20.06.2006 IT MI20061189
(43) Date of publication of application: 26.12.2007
(73) Proprietor: Eurosets S.r.l., 41036 Medolla (MO) (IT)
(72) Inventor: Ghelli, Nicola, 40018 S. Pietro In Casale (Prov. of Bologna) (IT); Costa Maianti, Edgardo, 41037 Mirandola (Prov. of Modena) (IT); Balanzoni, Roberto, 46020 San Giovanni Del Dosso (Prov. of Mantova) (IT); Balugani, Fabio, 41032 Cavezzo(Prov. of Modena) (IT)
(74) Representative: Alagem Modiano, Lara S.

(56) References cited:
- EP-A- 0 069 807
- EP-A1- 0 218 785
- EP-A1- 0 645 151
- GB-A- 1 434 967
- US-A- 5 695 489

## Description

The present invention relates to a venous bag in an extracorporeal blood circuit.

It is known that one of the devices that are present in extracorporeal blood circuits established during certain surgical procedures is a bag which is designed to contain blood which arrives from a line connected to a vein of the patient and is also connected, for optional additions, to a container, known as cardiotomy reservoir, which collects and filters the blood that arrives from the operating field; this bag is known as "venous bag".

Blood handling bags are disclosed by the documents EP-0645151A1 and EP-0218785A1.

Considering the presence, around the patient, of several complex devices which risk compromising or at least reducing the freedom of action of operators, it is readily evident that it is important to tend to reduce the volume of each individual device, and the aim of the present invention is to provide a venous bag which has a reduced space occupation during operation.

This aim is achieved by a venous bag in extracorporeal blood circuit, according to the invention, having the features set forth in claim 1.

Further advantageous features of the venous bag, according to the invention, are comprised in the dependent claims.

Further characteristics and advantages of the invention will become better apparent from the description of a preferred but not exclusive embodiment thereof, illustrated by way of nonlimiting example in the accompanying drawings, wherein:
Figure 1 is a front view of the bag in the emptying condition;
Figure 2 is a view of the bag in the expansion condition;

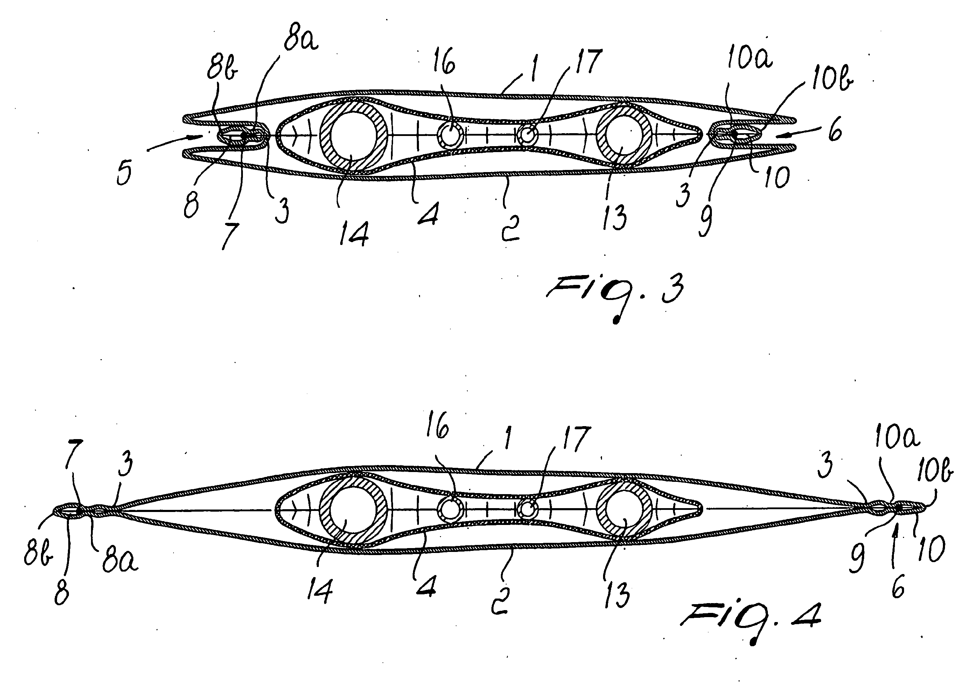

Figures 3 and 4 are sectional views, taken respectively along the line III-III of Figure 1 and along the line IV-IV of Figure 2.

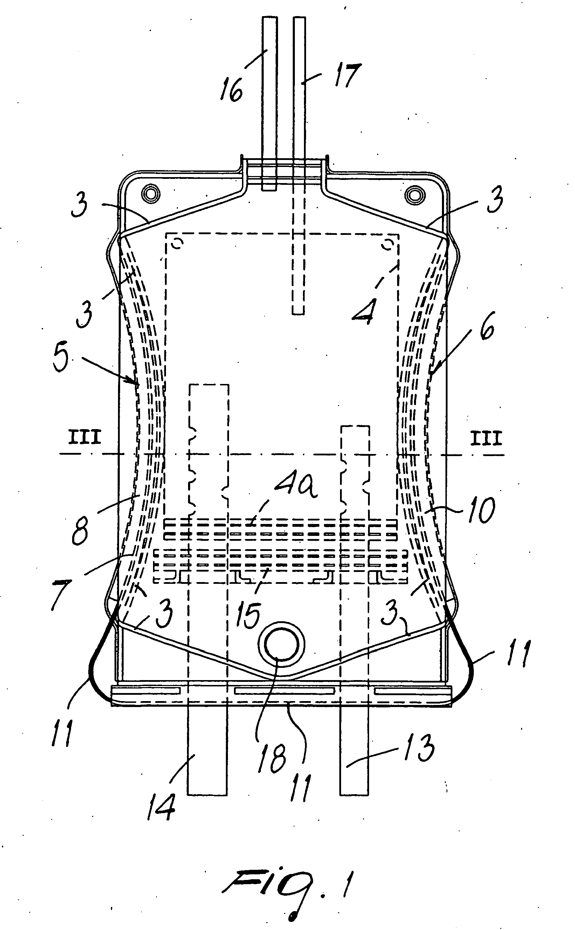

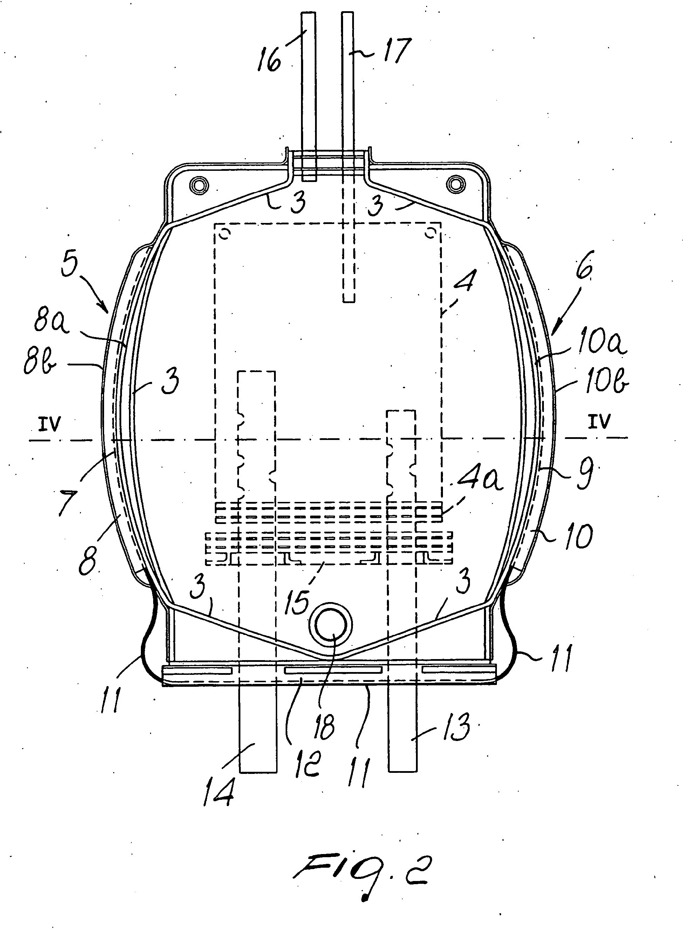

With reference to the figures, the bag according to the invention comprises two flat plastic sheets 1 and 2, which are connected one another by means of a thermal bonding 3 so as to define a closed containment volume for containing blood, which includes a filter 4 in the form of a bag which is heat-sealed at 4a and is designed to extract any air contained in the blood.
The bag comprises two sides provided by means of concertina portions 5 and 6, each provided with a portion of filament made of elastically deformable material which is accommodated in an appropriately provided channel which extends along the intermediate edge of the concertina portion, and thus the concertina portion 5 is provided with a portion of filament 7 inserted in a channel 8 delimited by heat seals 8a, 8b and the concertina portion 6 is provided with a portion of filament 9 inserted in a channel 10 delimited by heat seals 10a, 10b.
The portions of filament 7 and 9 are joined so as to form an assembly which is monolithic with a connecting portion 11 inserted in a channel 12 at the lower head of the bag at which tubes 13 and 14 enter; such tubes are heat-sealed to the bag at 15 and are connected to lines which arrive respectively from a cardiotomy reservoir and from a vein of the patient.
The upper head of the bag comprises drains 16 and 17, and the reference numeral 18 designates a coupling which is designed to be connected to a blood outflow line.
The presence of the portions of filament 7 and 9 ensures that every time emptying is performed from the expansion condition shown in Figure 2, the bag returns to the minimum-volume condition shown in Figure 1, and the correct operation of the filament is ensured by its monolithic configuration, which is obtained by means of the portion 11 for connecting the portions 7 and 9; it is in any case possible to ensure the correct operation of the portions of filament at the concertina portions, even if they are left independent from each other, by appropriately coupling them to the intermediate edge of the concertina portions, or by means of an external support.
The described invention is susceptible of numerous modifications and variations, all of which are within the scope of the appended claims.
Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A venous bag for an extracorporeal blood circuit, comprising two flat sheets (1,2) which are connected to one another (3) so as to determine a closed containment volume for containing blood, two concertina portions (5, 6) that connect two opposite sides of said sheets (1,2), and a filter (4) contained in said closed volume, the filter (4) for extracting any air contained in the blood, **characterized in that** it further comprises a filament (7, 9) made of elastically deformable material that is accommodated within a channel (8, 10) formed along the intermediate edge of the concertina portion (5, 6), the portions of filament (7, 9), that are present at said concertina portions (5, 6) being provided continuously by means of a connecting portion (11) which is accommodated within a channel (12) and connects said filament portions (7, 9) or independently of each other.

2. The bag according to claim 1, **characterized in that** said filter (4) has a bag shape, the venous bag comprising: a lower bag head provided with the channel (12) which is adapted to accommodate the connecting portion (11) and further provided with two blood intake tubes (13, 14) which are connectable to lines which arrive respectively from a cardiotomy reservoir and from the patient; an upper bag head provided with drains (16, 17) ; and a coupling (18) adapted to be connected to a blood outflow line being provided proximate to said lower bag head.

3. The bag according to claim 2, **characterized in that** said portions of filament (7, 9) and said connecting portion (11) form a monolithic configuration.

## Patentansprüche

1. Venöser Beutel für einen extrakorporalen Blutkreislauf, umfassend zwei flache Folien (1, 2), die miteinander verbunden sind (3), um so ein geschlossenes Aufnahmevolumen zur Aufnahme von Blut festzulegen, zwei Faltenbalgabschnitte (5, 6), die zwei gegenüberliegende Seiten der genannten Folien (1, 2) verbinden, sowie einen Filter (4), der in dem genannten geschlossenen Volumen enthalten ist, wobei der Filter (4) zum Entziehen jeglicher in dem Blut vorhandener Luft vorgesehen ist, **dadurch gekennzeichnet, dass** der Beutel ferner einen aus einem elastisch verformbaren Material hergestellten Draht (7, 9) umfasst, der in einem Kanal (8, 10) aufgenommen ist, der entlang der Zwischenkante des Faltenbalgabschnittes (5, 6) ausgebildet ist, wobei die Drahtabschnitte (7, 9), die an den genannten Faltenbalgabschnitten (5, 6) vorhanden sind, mit Hilfe eines Verbindungsabschnittes (11), der in einem Kanal (12) aufgenommen ist und die genannten Drahtabschnitte (7, 9) verbindet, durchgehend vorgesehen sind oder unabhängig voneinander sind.

2. Beutel nach Anspruch 1, **dadurch gekennzeichnet, dass** der genannte Filter (4) eine Beutelform hat, wobei der venöse Beutel umfasse einen unteren Beutelkopf, der mit dem Kanal (12) versehen ist, der dazu geeignet ist, den Verbindungsabschnitt (11) aufzunehmen, und ferner versehen mit zwei Blutansaugschläuchen (13, 14), die mit Leitungen verbunden werden können, die von einem Kardiotomiereservoir bzw. von dem Patienten kommen, einen oberen Beutelkopf, der mit Ableitungen (16, 17) versehen ist, und ein Anschlussstück (18), das dazu geeignet ist, mit einer Blutabflussleitung verbunden zu werden und das nahe dem genannten unteren Beutelkopf vorgesehen ist.

3. Beutel nach Anspruch 2, **dadurch gekennzeichnet, dass** die genannten Drahtabschnitte (7, 9) und der genannte Verbindungsabschnitt (11) eine monolithische Konfiguration bilden.

## Revendications

1. Sac veineux pour un circuit sanguin extracorporel, comprenant deux feuilles plates (1, 2) qui sont reliées l'une à l'autre (3) de façon à déterminer un volume de confinement clos pour contenir du sang, deux parties en accordéon (5, 6) qui relient deux côtés opposés desdites feuilles (1, 2), et un filtre (4) contenu dans ledit volume clos, le filtre (4) servant à extraire tout air contenu dans le sang, **caractérisé en ce qu'**il comprend de plus un filament (7, 9) réalisé en un matériau élastiquement déformable qui est reçu à l'intérieur d'un canal (8, 10) formé le long du bord intermédiaire des parties en accordéon (5, 6), les parties de filament (7, 9), qui sont présentes au niveau desdites parties en accordéon (5, 6) étant réalisées de façon continue à l'aide d'une partie de liaison (11) qui est reçue à l'intérieur d'un canal (12) et qui relie lesdites parties de filament (7, 9), ou indépendamment l'une de l'autre.

2. Sac selon la revendication 1, **caractérisé en ce que** ledit filtre (4) a une forme de sac, le sac veineux comprenant : une tête de sac inférieure pourvue du canal (12) qui est adaptée de façon à recevoir la partie de liaison (11), et pourvue de plus de deux tubes d'admission de sang (13, 14) qui peuvent être reliés à des lignes qui viennent, respectivement, d'un réservoir de cardiotomie et du patient ; une tête de sac supérieure pourvue de drains (16, 17) ; et un raccord (18) adapté de façon à être relié à une ligne d'écoulement de sortie de sang qui est disposé à proximité de ladite tête de sac inférieure.

3. Sac selon la revendication 2, **caractérisé en ce que** lesdites parties de filament (7, 9) et ladite partie de liaison (11) forment une configuration monolithique.
